# EUROPEAN PATENT APPLICATION

(11) **EP 2 832 396 A1**
(43) Date of publication of application: **04.02.2015**
(21) Application number: 12872536.3
(22) Date of filing: 21.12.2012
(51) Int. Cl.: A61M 25/10, A61L 29/00, A61M 25/14

(54) **BALLOON FOR CATHETER, AND BALLOON CATHETER**

(30) Priority: 28.03.2012 JP 2012073841
(71) Applicant: Terumo Kabushiki Kaisha, Tokyo 151-0072 (JP)
(72) Inventor: MAEDA, Naoyuki, B-3001 LEUVEN (BE); KOINUMA, Naoki, Fujinomiya-shi Shizuoka 418-0015 (JP); KAKIMOTO, Takuya, Fujinomiya-shi Shizuoka 418-0015 (JP)
(74) Representative: Casalonga
(86) International application number: PCT/JP2012/083333
(87) International publication number: WO 2013/145479

(57) **Abstract**

The present invention aims to provide a catheter balloon and a balloon catheter in which compliance characteristics of a membrane are improved in its entirety.

The present invention provides the catheter balloon including a membranous body that can dilate and contract by fluid supplied from a catheter. The membranous body includes an intermediate layer which is formed of a non-elastomer, an outer layer which is arranged on an outer surface of the intermediate layer and contains an elastomer, and an inner layer which is arranged on an inner surface of the intermediate layer and contains an elastomer. The average wall thickness of the intermediate layer is within 30% to 70% of the wall thickness of the balloon in its entirety.

## Description

### Technical Field

The present invention relates to a balloon and a balloon catheter.

Particularly, the present invention relates to a balloon and a balloon catheter inserted into body cavities.

### Background Art

A catheter equipped with a balloon (balloon catheter) is used for body organ expansion, which is performed for maintaining a body cavity space by means of placing a stent in a stenosed site in a blood vessel or a body cavity (a biological lumen such as the bile duct, the esophagus, the trachea, the urethra, and other organs). The catheter is also used for treating ischemic heart diseases or for urethral catheterization for patients having difficulty urinating.

Therefore, the catheter balloon requires characteristics such as (1) trackability (followability of a balloon with respect to a meandering blood vessel or body cavity), (2) passing characteristics with respect to a stenosed site such as a blood vessel, (3) dilatability of a stenosed site such as a calcified blood vessel, (4) diminished compliance (suitable non-extensibility not to cause any significant change in diameter of the balloon due to a small pressure change), (5) sufficient strength, pressure resistance, and the like for enduring an internal pressure or impact during dilation of the balloon.

Regarding the balloon used in a catheter, in a viewpoint of requiring safety to minimize a possibility of damage to a vascular tumor or a blood vessel as much as possible, pressure resistance, compliance characteristics, or the like are particularly important. However, when the pressure resistance is enhanced, the balloon itself is hardened resulting in a loss of flexibility of the balloon, thereby causing a problem of deterioration in passing characteristics of the balloon with respect to a stenosed site such as a blood vessel.

Here, as a technology focused on a balloon which has the high strength of a membrane and high flexibility in which the above-described problem is solved, JP-A-2003-144553 can be exemplified.

JP-A-2003-144553 discloses a balloon of which both of the ends of a straight tube portion have tapered shapes and of which thicknesses of the tapered portions throughout the tapered portion satisfy a relational expression (1) of the outer diameter of the straight tube portion, the wall thickness of the straight tube portion, a tapering angle, and a distance from a boundary between the straight tube portion and the tapered portion.

Moreover, according to PTL 1, it is possible to realize enhanced pressure resistance strength, a decrease in thickness, and flexibility of the balloon portion at the same time by changing the thickness of the membrane of the balloon in its entirety in accordance with the position of the balloon.

In addition, there has been a problem in that the balloon inflates exceedingly without being noticed by a doctor, since the doctor oneself cannot directly see the state of the balloon during dilation of the balloon.

Therefore, as a technology focused on the compliance in which the above-described problem is solved, there is JP-A-2006-110392.

JP-A-2006-110392 discloses a balloon which has semi-compliance characteristics and is formed of a polyurethane block copolymer exhibiting the compliance characteristics of 0.025 mm/atm to 0.045 mm/atm within an inflation pressure range of 6 atm to 19 atm, and a balloon which has non-compliance characteristics in an axial direction and is formed of a polyurethane block copolymer exhibiting the compliance characteristics of 0.01 mm/atm to 0.25 mm/atm within the inflation pressure range of 6 atm to 14 atm.

Moreover, since a balloon formed of a non-compliant material such as nylon needs to have a sufficiently large diameter even in a contracted state on account of the compliance characteristics thereof, a stent is prepared to introduce the balloon into the body of a patient in a folded state. However, the folded balloon has a problem in that uneven inflation of the stent may be caused.

The invention disclosed in JP-A-2006-110392 described above has solved the problem.

### Summary of Invention

### Technical Problem

A balloon disclosed in Fig. 1 or the examples of JP-A-2003-144553 is formed to have the wall thickness of the balloon in a tapered portion to be gradually thin from an opening portion side toward a straight tube portion side.

Therefore, the balloon disclosed in JP-A-2003-144553 has the straight tube portion which is relatively decreased in thickness so as to be able to satisfy to some extent passing characteristics with respect to a stenosed site such as a blood vessel.

However, since a raw material of the balloon actually used in the example is only a polyamide-based elastomer, pressure resistance is poorer than that of PET (the average bursting pressure is 21.3 atm in the example). In addition, there is a problem of a pin hole since the straight tube portion is decreased in thickness.

Moreover, since the thickness of the membrane in JP-A-2003-144553 is not even, there may be an occurrence of a significant change in diameter of the balloon due to a small pressure change, resulting in a problem of uncertainty in compliance characteristics.

Meanwhile, the invention in JP-A-2006-110392 focused on the compliance characteristics has been created in viewpoints of suppressing and preventing the folded balloon from causing uneven inflation of a stent. Therefore, in place of a non-compliant material such as polyamide exhibiting non-extensibility, the balloon is formed by using a one-layered membrane made of a polyurethane copolymer having semi-compliance characteristics.

However, since the outer wall that is to be in contact with a living body is made of a polyurethane copolymer, uneven inflation is suppressed, whereas the thickness of the membrane of the balloon is increased, thereby ruining trackability thereof.

In addition, since the semi-compliant material is used, non-extensibility is not secured during dilation of the balloon inside a living body, resulting in a problem of uncertainty in safety.

Accordingly, in order to solve the problems, the present invention aims to provide a balloon and a balloon catheter in which the compliance is improved and the balance is maintained between pressure resistance performance and passage performance.

### Solution to Problem

In order to achieve the above-described object, the present inventors have learned that in a case of considering a membrane of a balloon in its entirety, when characteristics of each layer configuring the membrane of the balloon or the thickness of the membrane is under predetermined conditions, the balloon exhibits the excellent compliance characteristics without ruining the balance between pressure resistance performance and passage performance, and thus, the present invention has been accomplished.

### Brief Description of Drawings

[Fig. 1] Fig. 1 is a schematic view illustrating an example of a catheter balloon according to the present invention.
[Fig. 2] Fig. 2 is a schematic view for illustrating a molding die of the catheter balloon according to the present invention.
[Fig. 3] Fig. 3 is a schematic view illustrating an example of a balloon catheter according to the present invention.
[Fig. 4] Fig. 4 is an experimental result describing a relationship between a certain pressure and an expansion rate of the pressure in examples of the present invention.

### Advantageous Effects of Invention

In a balloon according to the present invention, the balloon portion can be dramatically prevented from stretching caused by pressurization while maintaining pressure resistance performance and passage performance.

In the balloon according to the present invention, an outer wall thereof which comes into contact with a living body is formed of an elastomer, and thus, the balloon excels in trackability and passing characteristics with respect to a stenosed site such as a blood vessel.

### Best Mode for Carrying Out the Invention

Hereinafter, the present invention will be described below in detail.

In addition, the present application is based on Japanese Patent Application No. 2012-073841 filed March 28, 2012, the content of which is entirely incorporated herein as reference.

A first aspect of the present invention is a catheter balloon including a membranous body that can dilate and contract by fluid supplied from a catheter. In the catheter balloon, the membranous body includes an intermediate layer which contains a non-elastomer, an outer layer which is arranged on an outer surface of the intermediate layer and contains an elastomer, and an inner layer which is arranged on an inner surface of the intermediate layer and contains an elastomer. In the catheter balloon, the average wall thickness of the intermediate layer is within 30% to 70% of the average wall thickness of the balloon in its entirety.

In other words, as disclosed in JP-A-2003-144553 and JP-A-2006-110392 of the related art, when a catheter balloon is formed with a one-layered membrane or a two-layered membrane made of polyamide, a deviation of a dilation ratio in stretching occurs somehow between the inner side and the outer side of the tubular-shaped balloon during a producing process.

For further details, the inner side of the balloon is applied with a great force of stretching allowing a small range of stretching potential in the inner side portion thereof. In contrast, the outer side of the balloon is relatively less likely to be applied with the stretching force, thereby causing a difference in a stretching ratio between the outer side and the inner side of a balloon membrane.

As a result, it has been learned during the process of the present invention that the shape of the balloon is practically sustained by the inner side portion, and thus, the outside of the balloon does not contribute to pressure resistance thereof.

In addition, it has also been learned that when the inner side of the balloon membrane is applied with a great force of stretching, irregularity occurs in pressure resistance of the balloon itself, and thus, it is not possible to provide a balloon exhibiting stable pressure resistance.

Therefore, it has been learned that there can be provided a balloon excellent in compliance characteristics for dramatically suppressing the balloon portion from stretching caused by pressurization while maintaining pressure resistance performance and passage performance of the balloon in its entirety by containing an elastomer in an outer layer which is a region of the outer side of the balloon and does not contribute to pressure resistance, containing an elastomer in an inner layer which is a region of the inner side of the balloon where irregularity easily occurs in pressure resistance, and containing a non-elastomer contributing to pressure resistance in an intermediate layer which is a region between the outer layer and the inner layer.

In this manner, compared to that in the related art, since the compliance characteristics can be diminished by dramatically suppressing the balloon portion from stretching caused by pressurization while maintaining pressure resistance performance and passage performance, a lesion site can be securely widened, and a mucous membrane and an inner surface of a blood vessel can be suppressed from being damaged.

It is preferable for the average wall thickness of the intermediate layer according to the present invention to be within 30% to 70% of the average wall thickness of the balloon in its entirety and to be within 35% to 60% of the average wall thickness.

When the average wall thickness of the intermediate layer is less than 30% of the average wall thickness of the balloon in its entirety, there is a disadvantage in that pressure resistance is deteriorated. When the same exceeds 70% thereof, there is a disadvantage in that flexibility of the balloon portion is deteriorated.

In this specification, the term "compliance" indicating a degree of being easily expanded in diameter denotes an inclination of a compliance curve representing a relationship between an increase of the diameter and a pressure increase when an internal pressure is added thereto.

Meanwhile, an increasing ratio of a diameter when a certain internal pressure is added from a state under a nominal pressure is defined as "compliance under a certain pressure".

In addition, herein, the "nominal pressure" denotes a pressure necessary for the balloon to inflate to a fixed diameter.

The balloon according to the present invention preferably has an radial expansion rate from approximately 6% to approximately 16% within an inflation pressure range from a nominal pressure to the nominal pressure plus approximately 13 atm, and more preferably has the radial expansion rate from approximately 6% to approximately 12% within the inflation pressure range from the nominal pressure to the nominal pressure plus approximately 13 atm.

When the balloon has the radial expansion rate from approximately 6% to approximately 16% within the inflation pressure range from the nominal pressure to the nominal pressure plus approximately 13 atm (pressure range from nominal pressure to nominal pressure plus approximately 13 atm), the compliance becomes sufficiently diminished, thereby being able to be applied to widening of a lesion site and post-expansion of a stent without causing sudden inflation in the radial direction.

The expansion rate in this case is calculated by { (diameter of straight tube portion of balloon having nominal pressure + approximately 13 atm) - (diameter of straight tube portion of balloon having nominal pressure) } / (diameter of straight tube portion of balloon having nominal pressure) × 100 (%).

The balloon according to the present invention is preferably molded by inflating a tube which is obtained through three-layered coextrusion molding for molding a balloon to six times to nine times of an initial inner diameter in the radial direction and to twice to four times in an axial direction. The balloon is more preferably molded by inflating the tube which is obtained through the three-layered coextrusion molding for molding a balloon to seven times to eight times of an initial inner diameter in the radial direction and to 2.5 times to 3. 5 times in the axial direction.

The below-described method of producing a balloon will be described in detail. When the balloon has a tubular-shaped three-layered membrane (so-called three-layered parison) which is inflated to six times to nine times toward the outside in an axially perpendicular cross-sectional direction (or outside in radial direction) (or referred to as six times to nine times of stretching ratio in radial direction), the compliance can be decreased, thereby being preferable in the viewpoint of reducing a burden to a blood vessel during treatment.

In addition, when the balloon has the tubular-shaped three-layered membrane which is inflated to twice to four times toward the axial direction (or referred to as twice to four times of stretching ratio in axial direction), a balance between an orientation in the circumferential direction and an orientation in the axial direction becomes excellent, thereby being preferable in the viewpoint of an improvement of rupture resistance performance and safety.

The balloon according to the present invention preferably has the rupture resistance performance in the inflation pressure of the nominal pressure plus approximately 13 atm in a viewpoint of being applied to widening of a calcified lesion site or post-expansion of a stent, and more preferably has the rupture resistance performance within the inflation pressure range from the nominal pressure to the nominal pressure plus approximately 13 atm.

As a method of measuring rupture performance, a sample to be measured is immersed in warm water at the temperature of 37°C. When a step of applying a desired internal pressure for one minute and depressurizing to 1 atm is referred to as one cycle, the desired pressure is set as follows such as 1, 6, 8, 10,...20, 21,... atm to repeat the cycle. Then, higher pressures are applied until the balloon ruptures, thereby measuring the rupture resistance performance.

Hereinafter, the structure of the balloon according to the present invention will be described by using drawings, and then, the inner layer, the intermediate layer, and the outer layer which are constituent elements of the balloon will be described.

### "Balloon"

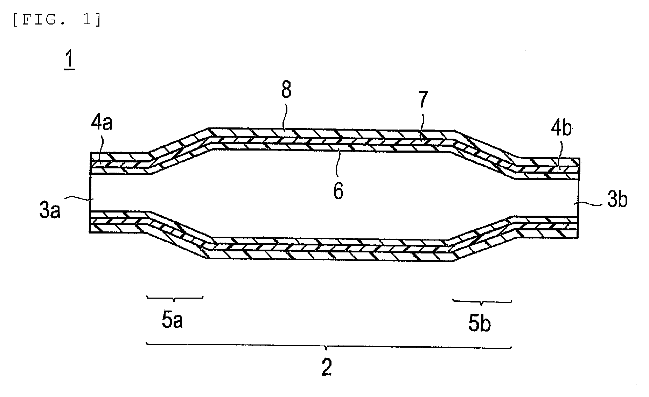

Fig. 1 is a cross-sectional view of an example of a structure in which the balloon according to the present invention is formed with a three-layered membranous body where an outer layer 8, an intermediate layer 7, and an inner layer 6 are sequentially laminated in the order thereof. The drawing is merely an example of the balloon, and the scope of the present invention is not limited thereto.

It is preferable for a balloon 1 according to the present invention to be a balloon made for catheters. It is preferable that the balloon 1 be configured to have a tubular membranous body 2 that can dilate and contract by fluid supplied from a catheter and connection portions 4a and 4b that extend from both of the ends of the membranous body in an axial direction and are connected to the catheter.

In addition, opening portions 3a and 3b through which a catheter is inserted are respectively formed in the connection portions 4a and 4b at both of the ends.

It is preferable for the opening portion 3b of one connection portion to have a diameter larger than that of the opening portion 3a of the other connection portion.

In addition, the balloon 1 includes a tubular portion which has a substantially uniform outer diameter for dilating a stenosed site of a lumen in the body such as a blood vessel, the ureter, and the bile duct.

As illustrated in Fig. 1, both of the end portions of the tubular membranous body may individually have a tapered shape (gradually narrowed shape).

Therefore, it is preferable for the balloon according to the present invention to have the tubular membranous body that includes tapered portions 5a and 5b of which both of the ends are individually formed to have the gradually narrowed shape, and the connection portions 4a and 4b that are respectively linked to the tapered portions 5a and 5b and are connected to the catheter which extends outwardly in the axial direction.

Furthermore, opening portions 3a and 3b through which the catheter is inserted are respectively formed in the connection portions at both of the ends.

When both of the end portions of the tubular membranous body individually have the tapered shape, a central portion of the tubular membranous body is a portion where the maximum diameter portion of the balloon continues. The tapered portions 5a and 5b are portions which are continued to the central portion of the tubular membranous body and change to continuously decrease in diameter toward the end portion thereof.

The connection portions 4a and 4b connected to the catheter are portions which respectively continue to the tapered portions 5a and 5b, and are formed to have substantially uniform inner diameters. The connection portions 4a and 4b serve as an attachment portion of the balloon with respect to the catheter, and the opening portions 3a and 3b are respectively formed therein.

Then, the tapered portions 5a and 5b and the connection portions 4a and 4b connected to the catheter are respectively positioned on both sides of the tubular membranous body of the balloon. Each of the tapered portions and each of the connection portions may have shapes different from each other.

As a size of the balloon according to the present invention, when the balloon is dilated by the nominal pressure, the outer diameter of the tubular membranous body is 1 mm to 6 mm, and is preferably 1.25 mm to 5.0 mm. The length of the tubular membranous body in a longitudinal axial direction is 5 mm to 40 mm, and is preferably 5 mm to 35 mm. The overall length (total length including tubular membranous body and connection portion in longitudinal axial direction) of the balloon is 8 mm to 50 mm, and is preferably 10 mm to 45 mm.

The axially perpendicular cross-sectional shape of the balloon according to the present invention may be a circular shape, an oval shape, a substantially oval shape, or a polygonal column shape, without being particularly limited.

The average thickness of the membrane at the time of contraction of the balloon according to the present invention is preferably 10 µm to 50 µm, and is more preferably 10 µm to 40 µm.

It is preferable for the balloon to have the average thickness of the membrane at the time of contraction of the balloon to be within the range of 10 µm to 50 µm, in the viewpoint of the trackability or the passing characteristics with respect to a stenosed site such as a blood vessel or a body cavity.

As described above, since the balloon of the present invention is formed with a membrane in which three layers are laminated, the connection portion formed at both of the ends of a membranous body 2 and is connected to the catheter as described in Fig. 1 may be a portion which is integrated (integrally molded) with the membranous body 2, or may be a portion which is separately provided to be bonded to a substantially cylindrical membrane body having a smaller diameter than that of the tubular membranous body 2.

Therefore, the average thickness of the membrane of the connection portion according to the present invention in a normal state is preferably 20 µm to 200 µm, and is more preferably 20 µm to 180 µm.

The balloon according to the present invention includes a membranous body which can dilate and contract by fluid supplied from a catheter, and thus, it is preferable for the balloon to be able to be folded, and to be able to be in a state of being folded around the outer circumference of the tube of the catheter body when in a contracted state.

Moreover, the surface of the outer layer and/or the inner layer according to the present invention may be coated with a biocompatible material or an antithrombotic material as necessary.

As the biocompatible material or the antithrombotic material, various known polymers can be used alone or a mixture thereof can be used. For example, a natural polymer (collagen, gelatin, chitin, chitosan, cellulose, polyaspartic acid, polyglutamic acid, polylysine, casein, and the like), and a synthetic polymer (a phosphatide polymer and a methacryloyloxyethyl phosphorylcholin (MPC) block polymer having a phosphoric acid group on the side chain thereof, polyhydroxyethyl methacrylate, a hydroxyethyl methacrylate-styrene copolymer (for example, a HEMA-St-HEMA block copolymer), polymethyl methacrylate, polylactic acid, polyglycolic acid, a lactic acid-glycolic acid copolymer, polyethylene, and polypropylene) can be preferably used.

In addition, in order to make it easy to insert the catheter balloon according to the present invention into a blood vessel as well as a guide catheter, it is preferable to perform processing on the outer surface of the balloon or the membranous body so as to exhibit lubricity when coming into contact with blood and the like.

As such processing, a method of coating the surface with a hydrophilic resin such as poly (2-hydroxyethyl methacrylate), polyhydroxyethyl acrylate, hydroxypropyl cellulose, a methyl vinyl ether-maleic anhydride copolymer, polyethylene glycol, polyacrylamide, polyvinyl pyrrolidone, and a random or block copolymer of dimethylacrylamide-glycidyl methacrylate; and a method of fixing the same onto the surface can be exemplified.

It is preferable for the outer layer and the inner layer to be formed in close contact with the surface of the intermediate layer, and it is preferable for the outer layer and the inner layer to be formed in close contact with the entire surface of the intermediate layer.

In this manner, it is possible to provide a balloon maintaining pressure resistance and passing characteristics.

Hereinafter, the inner layer, the outer layer, and the intermediate layer which are constituent elements of the balloon according to the present invention will be described.

### (Intermediate Layer)

The intermediate layer according to the present invention is made of a non-elastomer and occupies 30% to 70% of the average thickness of the membrane of the balloon in its entirety.

In other words, a region between an outer layer and an inner layer is formed to be the intermediate layer which is made of a non-elastomer contributing to pressure resistance. In a cross section which is obtained by cutting a middle portion of the balloon according to the present invention in the axially perpendicular direction, when considering an axis in which a position on an inner surface of the balloon (inner surface of inner layer) is set to 0 and a position on an outer surface of the balloon (outer surface of the outer layer) towards the outside in a radiation direction is set to 1, the region within 0.1 or 0.2 from the inner surface (0) is a region in which a stretching pressure is likely to be applied, and the region within 0.5 or 0.8 from the outer surface (1) is a region in which the stretching pressure is unlikely to be applied.

Therefore, it is preferable to set the inner layer to be the region within 0.1 or 0.2 from the inner surface (0), the outer layer to be within 0.5 or 0.8 from the outer surface (1), and the intermediate layer to be the remaining region (preferably 0.1 to 0.8, more preferably 0.2 to 0.5).

The region occupied by the intermediate layer may be changed in accordance with the rupture resistance performance and the passing performance required.

The average thickness of the intermediate layer in the membranous body configuring the balloon according to the present invention is preferably 5 µm to 20 µm.

The thickness of the intermediate layer may be a suitable thickness in consideration of the rupture resistance performance and the passing characteristics required with respect to the diameter of the balloon.

As a method of measuring the average thickness of the intermediate layer in the membranous body configuring the balloon according to the present invention, a calculation is made by converting from the design dimensions of an original tube and the thickness of the membrane of the balloon.

When it is said that the intermediate layer according to the present invention is formed of the non-elastomer, it denotes that the intermediate layer according to the present invention does not contain any elastomer, and it is preferable for the intermediate layer to be formed of polyamide.

In other words, it is preferable for the intermediate layer according to the present invention to contain polyamide. The intermediate layer may contain a known additive or a contrast medium for X-rays and the like as necessary, and may be constituted only with polyamide. When the intermediate layer contains polyamide within a range of 1% by weight to 100% by weight, pressure resistance strength or compliance required for a catheter balloon can be secured.

Polyamide which can be preferably used for the intermediate layer according to the present invention is not particularly limited as long as it has an acid amide bond (-CO-NH-) on a main chain. Generally, the polyamide is produced by polymerization (homopolymerization) of lactam or amino acid having a cyclic structure, or condensation polymerization of dicarboxylic acid and diamine.

Therefore, it is preferable to use homopolyamide as the polyamide.

As a monomer which can be homopolymerized, ε-caprolactam, aminocaproic acid, enantholactam, 7-aminoheptanoic acid, 11-aminoundecanoic acid, 12-aminododecanoic acid, 9-aminononanoic acid, and peperidone can be exemplified.

In addition, as the dicarboxylic acid to be subjected to condensation, polymerization together with diamine, adipic acid, sebacic acid, dodecanedicarboxylic acid, glutaric acid, terephtalic acid, 2-methylterephthalic acid, isophthalic acid, and naphthalene dicarboxylic acid can be exemplified.

As the diamine, tetramethylenediamine, hexamethylenediamine, nonamethylenediamine, decamethylenediamine, undecamethylenediamine, dodecamethylenediamine, paraphenylenediamine, and metaphenylenediamine can be exemplified.

In addition, as the polyamide, nylon 4, 6, 7, 8, 11, 12, 6.6, 6.9, 6.10, 6.11, 6.12, 6T, 6/6.6, 6/12, 6/6T, and 6T/6I can be exemplified.

Moreover, a terminal of the polyamide may be sealed with a carboxyl group, an amino group, or the like.

The polyamide resin can be adopted in one type alone, or in combination of two or more types.

It is preferable for the polyamide according to the present invention to be nylon 11 or nylon 12 from among those described above.

The weight average molecular weight of the polyamide according to the present invention is preferably 2.0 × 10⁴ to 5.0 × 10⁴, more preferably 3.0 × 10⁴ to 5.0 × 10⁴, and even more preferably 4.0 × 10⁴ to 5.0 × 10⁴.

The weight average molecular weight of the polyamide according to the present invention can be measured by a known method such as mass spectrometry, a light scattering method, liquid chromatography, and gas chromatography. In the present specification, a molecular weight which is measured by gel permeation chromatography is used.

As an additive to be contained in the intermediate layer as necessary, a higher alcohol, hydroxybenzoic acid ester, and aromatic sulfonamide can be exemplified, but the present invention is not limited thereto.

As the additive to be contained in the intermediate layer as necessary in the present invention, without being particularly limited as long as the additive is radiopaque with respect to radiation rays, a known radiopaque substance can be used.

Specifically, iodine, barium, bismuth, boron, bromine, calcium, gold, platinum, silver, iron, manganese, nickel, gadolinium, dysprosium, tungsten, tantalum, and a compound of these such as barium sulfate, as well as a solution/dispersion liquid of these (for example, physiological saline); amidotrizoic acid (3,5-diacetamino-2,4,6-triidobenzoic acid), amidotrizoate sodium meglumine, amidotrizoate meglumine, iothalamate sodium, iothalamate meglumine, iotroxate meglumine, iotrolan, ioxaglic acid, ioxalan, iopamidol, iopromide, iohexol, ioversol, and iomeprol; and iodized poppy oil fatty acid ethyl ester (for example, Lipiodol™, that is poppy seed oil in which carbon atoms are iodized) can be exemplified.

Those radiopaque substances may be used in one type alone, or may be used in a form of a mixture of two or more types thereof.

In addition, a contrast medium layer having the above-described contrast medium as a base medium may be further laminated on the membranous body.

In this manner, the degree of dilation of the balloon can be checked through radioscopy, and thus, the position of the balloon can be securely and easily confirmed.

In the axially perpendicular cross section of the middle portion in the axial direction of the balloon according to the present invention, the cross-sectional ratio of the intermediate layer is preferably 30% to 70%, and is more preferably 35% to 60%.

### (Outer Layer)

The outer layer according to the present invention contains an elastomer.

In addition, the outer layer containing an elastomer may contain the elastomer of 1% by weight to 100% by weight, may contain a known additive or a contrast medium for X-rays as necessary, or may be constituted only with an elastomer.

If the outer layer containing an elastomer is formed on the outermost surface, when the balloon is mounted on the catheter to be inserted into a body, it becomes excellent in passing characteristics with respect to the inside of a blood vessel or a body cavity for being flexible.

The average thickness of the outer layer in the membranous body configuring the balloon according to the present invention is preferably 5 µm to 15 µm, and is more preferably 5 µm to 10 µm.

When the average thickness is within the range of 5 µm to 15 µm, an improvement can be achieved in friction resistance with respect to a hard component such as a calcified lesion site, thereby making it possible to apply treatment more safely.

The material of the outer layer according to the present invention may be the same as the material of the inner layer according to the present invention, and the elastomer in the outer layer according to the present invention may be the same as the elastomer in the inner layer according to the present invention.

It is preferable for the elastomer contained in the outer layer according to the present invention to be a polyamide elastomer having a sufficient adhesive property with respect to the polyamide in the intermediate layer in order to prevent delamination therebetween.

It is preferable for the polyamide elastomer to be a polyamide block copolymer, and is more preferable to be a diblock copolymer having a hard segment and a soft segment.

As the diblock copolymer, a block copolymer of polyamide (hard segment) and polyether (soft segment) can be exemplified, and specifically a block copolymer of nylon 11 and polytetramethylene glycol, and a block copolymer of nylon 12 and polytetramethylene glycol can be exemplified.

The shore D hardness of the polyamide elastomer according to the present invention is preferably 40 to 75, and more preferably 55 to 65.

The tensile modulus of the polyamide elastomer according to the present invention is preferably 100 MPa to 500 MPa, and more preferably 100 MPa to 250 MPa.

The passing characteristics can be expected to be improved by using a softer polyamide elastomer.

It is preferable for the polyamide elastomer according to the present invention to have a block copolymer represented by the following Chemical Formula (1) or (2) in a polymer chain. 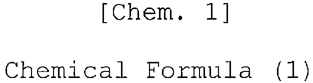 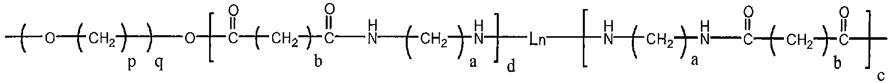 (In Chemical Formula (1), a is an integer within 4 to 12, b is an integer within 4 to 10, c is an integer within 0 to 100, d is an integer within 0 to 100, p is an integer within 2 to 4, and q is an integer within 1 to 100. Ln is a linker region of -C (O)-R-O-C (O)-, and R is an alkylene group having 2 to 12 methylene groups.) 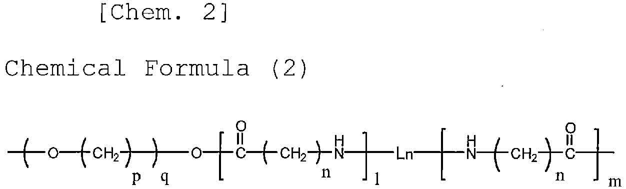 (In Chemical Formula (2), n is an integer within 5 to 11, 1 is an integer within 0 to 100, m is an integer within 0 to 100, p is an integer within 2 to 4, and q is an integer within 1 to 100. Ln is a linker region of -C(O)-R-O-C(O)-, and R is an alkylene group having 2 to 12 methylene groups.)

In other words, the polyamide elastomer according to the present invention may be the polyamide block copolymer itself in Chemical Formula (1) or Chemical Formula (2), or an elastomer which is obtained by further polymerizing the polyamide block copolymer in Chemical Formula (1) or Chemical Formula (2) through melt polymerization. However, it is preferable for the polyamide elastomer according to the present invention to be the elastomer which is obtained by further polymerizing the polyamide block copolymer in Chemical Formula (1) or Chemical Formula (2) through the melt polymerization.

Accordingly, when the polyamide elastomer is further polymerized through the melt polymerization, the polyamide block copolymer in Chemical Formula (1) or Chemical Formula (2) is formed to be a so-called "repeating unit".

In addition, R in Chemical Formula (1) and Chemical Formula (2) is not particularly limited so that R may be straight-chained, branched, or cyclic as the alkylene group having 2 to 12 methylene groups. Specifically, a tetramethylene group, a 2-methylpropylene group, a 1,1-dimethylethylene group, an n-pentylene group, an n-hexylene group, an n-nonylene group, a 1-methyloctylene group, a 6-methyloctylene group, a 1-ethylheptylene group, a 1-(n-butyl)pentylene group, a 4-methyl-1-(n-propyl)pentylene group, a 1,5,5-trimethylhexylene group, a 1,1,5-trimethylhexylene group, an n-decylene group, a 1-methylnonylene group, a 1-ethyloctylene group, a 1-(n-butyl)hexylene group, a 1,1-dimethyloctylene group, a 3,7-dimethyloctylene group, an n-undecylene group, and a 1-methyldecylene group can be exemplified.

A further polymerized polyamide elastomer can be obtained by performing melt polymerization onto the polyamide elastomer of which both the terminals are not sealed.

The melt polymerization can be performed by heating a polyamide elastomer at 220°C to 250°C for a certain period (12 to 72 hours) under vacuum formed by a vacuum pump (GCD136XN manufactured by ULVAC KIKO, Inc.) using a vacuum drier (VOS301SD manufactured by EYELA) equipped with a cooling function (cooling machine; UT-4000L manufactured by EYELA).

When the polyamide block copolymer in Chemical Formula (1) or Chemical Formula (2) is used for the outer layer according to the present invention, the polyamide block copolymer in Chemical Formula (1) or Chemical Formula (2) may be adopted in one type alone, or in combination of two types.

The polyamide elastomer according to the present invention may be synthesized, or may be purchased from commercially available products. As the polyamide elastomer usable in the present invention, ELG5660 (manufactured by EMS-GRIVORY, trade name: Grilflex ELG5660), ELG6260 (manufactured by EMS-GRIVORY, trade name: Grilflex ELG6260), a high-molecular weight elastomer (having a melt viscosity of 1,260 Pa·s to 3,489 Pa·s) obtained by performing melt polymerization on the ELG5660, and a high-molecular weight elastomer (having a melt viscosity of 5,282 Pa·s to 7, 391 Pa·s) obtained by performing melt polymerization on the ELG6260 can be exemplified.

In addition, the terminal of the polyamide elastomer according to the present invention may be sealed with a carboxyl group, an amino group, and the like.

The melt viscosity of the polyamide elastomer according to the present invention is preferably 500 Pa·s or higher, and more preferably 500 Pa·s to 20,000 Pa·s.

This is because stretching caused by pressurization is suppressed so that the balloon exhibits lower compliance in its entirety.

In the present invention, the melt viscosity is measured by using a flow tester "CFT-500D manufactured by Shimadzu Corporation".

The additives or the radiopaque substances which may be contained in the outer layer as necessary are the same as those of the intermediate layer. Accordingly, description thereof will not be repeated herein.

In the axially perpendicular cross section of the middle portion in the axial direction of the balloon according to the present invention, the cross-sectional ratio of the outer layer is preferably 20% to 50%, and is more preferably 25% to 50%.

Being within the range, it is possible to achieve both the rupture resistance performance and widening performance for a hardened lesion site such as a calcified lesion site.

### (Inner Layer)

The inner layer according to the present invention contains an elastomer.

In addition, the inner layer containing an elastomer may contain the elastomer of 1% by weight to 100% by weight, may contain a known additive or a contrast medium for X-rays as necessary, or may be constituted only with an elastomer.

If the inner layer containing an elastomer is formed on the innermost side, it is possible to provide a catheter balloon exhibiting stable pressure resistance and it is possible to grant flexibility to the balloon in its entirety, it becomes excellent in passing characteristics with respect to the inside of a blood vessel or a body cavity.

The average thickness of the inner layer in the membranous body configuring the balloon according to the present invention is preferably 0.1 µm to 10 µm, and is more preferably 0.1 µm to 7 µm.

Since the balance between flexibility of the balloon in its entirety and diminished compliance characteristics can be achieved, it is preferable to have the average thickness within 0.1 µm to 10 µm.

It is preferable that the elastomer contained in the inner layer according to the present invention be a polyamide elastomer which has a favorable adhesive property with respect to the intermediate layer and does not cause any delamination therebetween.

As the polyamide elastomer contained in the inner layer according to the present invention, the same material as the polyamide elastomer in the outer layer can be used, thereby omitting the description for the polyamide elastomer herein.

From the above description, as a preferable aspect of the balloon according to the present invention, it is preferable for the balloon to be formed with a membranous body in which an inner layer formed of a polyamide elastomer is provided on the innermost side, an intermediate layer formed of a polyamide is laminated on the surface of the inner layer, and an outer layer formed of a polyamide elastomer is further provided on the outer side of the intermediate layer.

Accordingly, it is possible to provide a balloon exhibiting the excellent compliance characteristics without ruining the balance between pressure resistance performance and passage performance.

### "Method of Producing Balloon"

Hereinafter, a preferable embodiment for a method of producing the balloon according to the present invention will be described.

It is preferable for the method of producing the balloon according to the present invention to include a step (1) in which coextrusion is performed by a wire coating method as general means, an intermediate layer is formed of a non-elastomer, and a three-layered polymer tube (parison) having an inner layer and an outer layer being formed of an elastomer is formed; a step (2) in which the parison is stretched in the axial direction at a temperature range from a secondary transition temperature to a primary transition temperature of both the polymers, and the stretched parison is biaxially stretched by performing inflating in the radial direction; a step (3) in which the inflated parison is cooled to a temperature equal to or lower than the secondary transition temperature of both the polymers, the cooled parison is contracted to form a biaxially stretched balloon including a tubular membranous body that has a substantially uniform inner diameter, tapered portions that are respectively provided in the front and back of the membranous body, and connection portions that are respectively provided in the front and back of the tapered portions and connected to a catheter; and a step (4) in which the thicknesses of the tapered portions are decreased by re-stretching the tapered portions of the biaxially stretched balloon as necessary, the re-stretched balloon is inflated, after the balloon is heated to a temperature equal to or higher than the secondary transition temperature of the polymers while maintaining the inflated state, and then, the balloon is cooled to a temperature equal to or lower than the secondary transition temperature of the polymers.

The present invention has focused on the thickness of each layer configuring the membrane of the balloon, and it is learned that when generally producing a balloon by a biaxial stretching method, there is an occurrence of irregularity in pressure resistance of the balloon itself as a product or in pressure resistance for each product due to deviation of a pressure (in radial direction and axial direction of balloon) which is applied when performing the stretching during the producing steps.

As mentioned in the examples to be described below, it is learned that if each layer of the membranous body which forms the balloon does not have a wall thickness within a desired range, the compliance characteristics of the balloon is diminished and the rupture pressure of the balloon or the flexibility of the balloon is ruined.

However, on account of following conditions of steps (1) to (3), particularly, by having a blowing temperature of 70°C to 100°C, an annealing temperature of 100°C to 130°C, blowing and annealing pressures of 3.0 MPa to 4.5 MPa, a stretching ratio in the radial direction of six times to nine times, and a stretching ratio in the axial direction of twice to four times, it is possible to produce a balloon having a membranous body provided with layers each of which a thickness of the membrane is within a desired range. As a result, it is possible to provide a balloon which excels in compliance characteristics, flexibility, and rupture pressure.

In addition, it is preferable for the inner layer, the outer layer, and the intermediate layer to be made of the same-based materials.

In the embodiments, although the polyamide-based material is used for all layers, for example, it is possible to use a polyurethane-based material for all layers such as the intermediate layer formed of a polyurethane non-elastomer and the inner and outer layers formed of a polyurethane-based elastomer.

It is possible to acquire a balloon having high adhesive strength between layers by using the same-based material.

Hereinafter, each of the steps (1) to (4) will be described in detail.

### Step (1)

The step (1) in which a tubular-shaped parison (hereinafter, also referred to as tube or parison) is formed of a stretchable polymer can be performed by using a general-purpose extruder equipped with a dice.

As molding polymers, elastomers are used as the materials for the outer layer and the inner layer, and a non-elastomer is used as the material for the intermediate layer.

Each of the molding polymers is heated and melted at a temperature of 150°C to 270°C in an extruder so as to cause the elastomer to be 40 parts by weight to 240 parts by weight with respect to the non-elastomer of 100 parts by weight. Then, the polymers are subjected to the coextrusion from the dice, thereby forming a parison 27.

The ratio between the intermediate layer corresponding to the non-elastomer layer and the inner layer and outer layer corresponding to the elastomer layers is controlled, thereby determining the thickness of the membrane of the intermediate layer of the final mold for the balloon.

The temperature of the extrusion molding at this time is not particularly limited as long as the polymers can be melted thereat. However, the temperature is preferably 160°C to 260°C, and more preferably 170°C to 260°C while an extrusion pressure is 0.5 MPa to 10.0 MPa.

### Step (2)

The step (2) in which the parison is inflated in the radial direction to perform the biaxial stretching can be carried out by adopting a molding die for a balloon.

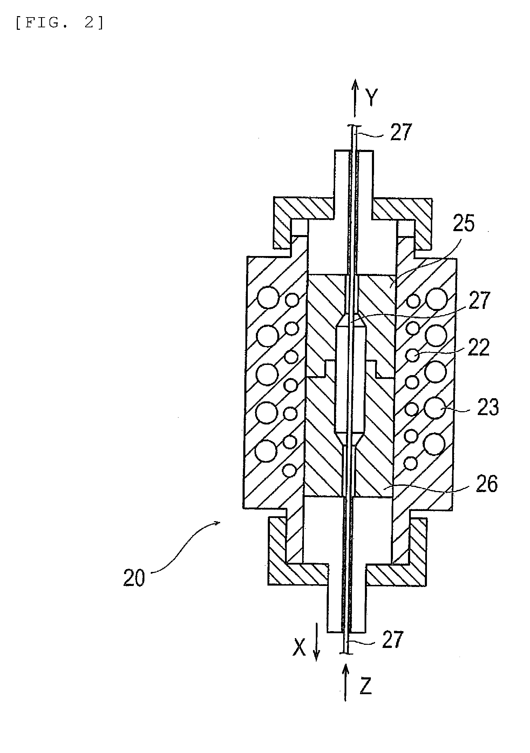

Specifically, the tubular-shaped parison 27 is inserted into a die 20 which is illustrated as an example in Fig. 2, and one end of the tube 27 is blocked.

The blocking is performed by heating and melting, high frequency sealing, or using of forceps and the like.

Fig. 2 is a cross-sectional view of the molding die 20 for a balloon.

The die 20 includes heaters 22 as heating means and cooling tubes 23 as cooling means.

Then, the die 20 is configured to have separable molds 25 and 26. The shape of the inner surface which is formed when the separable molds 25 and 26 are combined together becomes the basic shape of the outer surface of the balloon to be formed.

Then, as illustrated in Fig. 2, the heaters 22 are operated so as to heat the tube 27 corresponding to the portion for forming a balloon 1 at a temperature (70°C to 100°C) which slightly exceeds the temperature range of 30°C to 60°C of glassy-transition temperature for the polymers (non-elastomer and elastomer forming tube 27).

While the tube 27 is kept in a heated state, the tube 27 is stretched in directions of the arrows X and Y. Moreover, gas is supplied to the inside of the tube 27 from a direction of the arrow Z in a pressurized state. Then, the tube 27 which is the portion heated inside the die 20 is brought into close contact with the inner wall surfaces of the separable molds 25 and 26.

Here, it is preferable for the stretching ratio to be applied six times to nine times in the axially perpendicular cross-sectional direction (radial direction of balloon) of the balloon, and twice to four times in the axial direction (axially longitudinal direction of balloon).

The degree of a stretching distance in an XY-direction is determined by a pressure added from a Z-direction at this time.

When the internal pressure is too low, the balloon cannot be molded unless the stretching distance is extended, and when the internal pressure is too high, the balloon is molded before stretched in the axial direction.

It is preferable to apply a pressure to the inside of the tube so as to be able to settle the stretching distance in the axial direction within the extent of the above-described stretching ratio.

### Step (3)

The step (3) to form a balloon including a tubular membranous body, tapered portions that are respectively provided in the front and back of the membranous body, and connection portions that are respectively provided in the front and back of the tapered portions and connected to a catheter is formed by stretching processing.

Specifically, a coolant is circulated inside the cooling tubes 23 to cool the tube 27 to a temperature equal to or lower than the secondary transition temperature.

In addition, regarding the cooling, natural cooling may be adopted by being simply left behind in place of circulating the coolant.

Thereafter, the inside of the tube 27 is under the normal pressure, and then, the tube 27 is drawn out from the inside of the die 20.

Then, the tube 27 is cut in the distal end portion and a rear end portion thereof, thereby forming a basic shape of the balloon as illustrated in Fig. 1.

The stretching processing may be performed twice or more to form a balloon having a desired thickness.

### Step (4)

The step (4) in which the thickness is decreased by re-stretching the balloon, and after the balloon is heated to a temperature equal to or higher than the secondary transition temperature of the polymers, the balloon is cooled to the temperature equal to or lower than the secondary transition temperature of the polymers may be provided after the step (3) as necessary.

Specifically, the tapered portions 5a and 5b of the biaxially stretched balloon may be re-stretched to decrease the thickness of the tapered portion, the re-stretched balloon may be inflated, after the balloon is heated to a temperature equal to or higher than the secondary transition temperature of the polymers while maintaining the inflated state as in the step (2), and then, the balloon may be cooled to the temperature equal to or lower than the secondary transition temperature of the polymers.

### "Balloon Catheter"

The balloon catheter according to the present invention is a balloon catheter for widening a blood vessel including an inner tube that has a first lumen of which the distal end is open; an outer tube that is provided coaxially with the inner tube, has the distal end in a position which recedes for a predetermined length from the distal end of the inner tube, and forms a second lumen between the an outer surface of the inner tube and itself; and a foldable balloon of which a distal end portion is fixed to the inner tube, a proximal end portion is fixed to the outer tube, and the inside thereof communicates with the second lumen. In the balloon catheter, it is preferable for the balloon to be the balloon according to the present invention.

As a suitable embodiment of the catheter balloon according to the present invention, hereinafter, the balloon catheter according to the present invention will be described with reference to the drawings. However, the scope of the present invention is not limited to the following embodiments or drawings only.

In the description of drawings, the same numeral/sign is applied to the same element, thereby omitting a repeated description.

In addition, the dimensional ratio of the drawings is exaggerated for the convenience of description and may be different from the actual ratio in some cases.

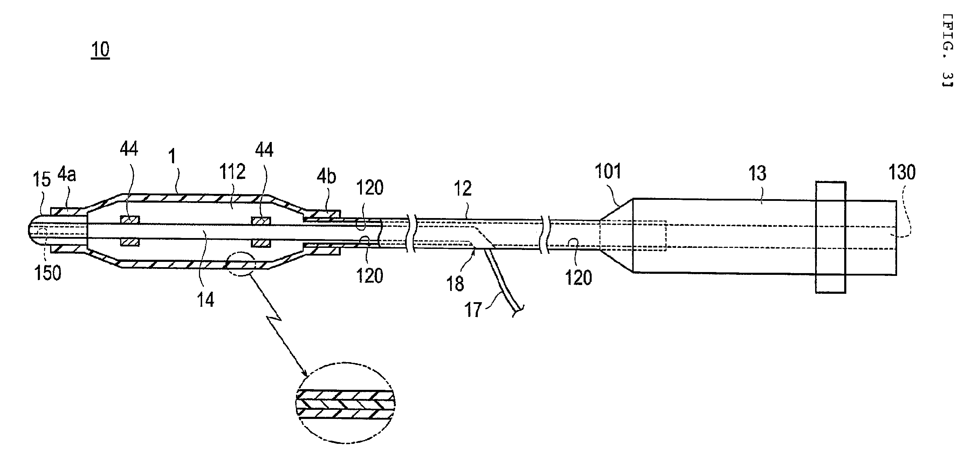

Fig. 3 is a schematic view illustrating an example of the balloon catheter according to the present invention.

The structure of the balloon catheter 10 according to the present invention will be described.

The balloon catheter 10 includes an inner tube 14 that has a first lumen 150 of which the distal end is open; an outer tube 12 that is provided coaxially with the inner tube 14 in a position which recedes to the proximal end side for a predetermined length from the distal end of the inner tube 14, and forms a second lumen 120 between the an outer surface of the inner tube 14 and itself; and a foldable balloon 1 which has a connection portion (distal end portion side of balloon) 4a connected to the catheter and a connection portion (proximal end portion side of balloon) 4b connected to the catheter, in which the connection portion 4b is fixed to the outer tube 12 and the connection portion 4a is fixed to the inner tube 14, and which communicates with the second lumen 120 in the vicinity of the proximal end portion.

In addition, it is preferable for the balloon catheter 10 to be provided with a hub 13 including an opening portion which communicates with the second lumen.

Moreover, the balloon 1 covers the vicinity of the proximal end portion of the outer tube 12, and the inside 112 of the balloon 1 communicates with the second lumen 120.

In Fig. 3, an embodiment of the balloon catheter 10 according to the present invention has a catheter body 101 that includes the elongated outer tube 12 which is able to transport fluid, the balloon 1 that is connected to the distal end of the catheter body 101, and the hub 13 that is mounted on the proximal end of the catheter body 101.

In addition, the balloon catheter 10 has an inner tube 14 that passes through the inside of the second lumen 120 formed inside the outer tube 12, and a distal end member 15 that is provided at the distal end of the inner tube 14.

The distal end indicates an end portion which is positioned on a side to be inserted into a blood vessel at the time of use, and the proximal end indicates an end portion positioned on a side of an operator who operates the balloon catheter 10 at the time of use.

Fig. 3 illustrates a rapid exchange-type catheter in which the catheter is formed to have a single lumen on the side of the proximal end portion and there is provided a wire port into which a guide wire can be inserted between the distal end and the proximal end thereof. However, the catheter may be an over-the-wire type in which the catheter is formed to have a coaxial double lumen on the side of the proximal end portion and the inner tube extends to the hub.

The balloon catheter 10 is an example applied to a vasodilating catheter. The balloon and the balloon catheter according to the present invention can also be applied to other catheters such as a urethral catheter.

As the fluid supplied to the balloon from the catheter, known fluids such as a contrast medium, helium gas, a physiological saline, CO₂ gas, O₂ gas, N₂ gas, and air can be exemplified.

The balloon catheter 10 according to the present invention is configured to have the catheter body 101 that includes the inner tube 14 and the outer tube 12, the hub 13, and the balloon 1.

The inner tube 14 has a first lumen 150 (outer lumen on the inner side) having an opened distal end.

The first lumen 150 is a lumen for inserting a guide wire therethrough and communicates with a wire port 18 which is an opening portion forming a guide wire port.

Then, a guide wire 17 can be inserted through the wire port 18.

The outer diameter of the inner tube 14 is 0.30 mm to 2.50 mm and preferably 0.40 mm to 2.00 mm, and the inner diameter thereof is 0.20 mm to 2.35 mm and preferably 0.25 mm to 1.70 mm.

It is preferable that the material for forming the inner tube 24 be flexible to some extent. For example, polyolefin such as polyethylene, polypropylene, an ethylene-propylene copolymer, and an ethylene-vinyl acetate copolymer; and a thermoplastic resin such as polyvinyl chloride, polyurethane, polyamide, a polyamide elastomer, and a polyester elastomer can be used.

The inner tube 14 is inserted through the inside of the outer tube 12. The distal end of the outer tube 12 is provided in a position which slightly recedes from the distal end of the inner tube. The inner surface of the outer tube 12 and the outer surface of the inner tube 14 form the second lumen 120.

Accordingly, the second lumen 120 can be a lumen having a sufficient volume.

Moreover, the distal end of the second lumen 120 communicates with the inside of the above-described balloon 1 at the rear end portion thereof. The rear end of the second lumen 120 communicates with an opening portion 130 of the hub 13 which forms an injection port for injecting fluid (for example, contrast medium, helium gas, a physiological saline, CO₂ gas, or O₂ gas) for inflating the balloon.

The outer diameter of the outer tube 12 is 0.50 mm to 4.30 mm and preferably 0.60 mm to 4.00 mm, and the inner diameter thereof is 0.40 mm to 3.80 mm and preferably 0.50 mm to 3.00 mm.

In addition, the above-described radiopaque substances may be injected into the balloon during dilation of the balloon as necessary.

It is preferable that the material for forming the outer tube 12 be flexible to some extent. For example, polyolefin such as polyethylene, polypropylene, an ethylene-propylene copolymer, and an ethylene-vinyl acetate copolymer; and a thermoplastic resin such as polyvinyl chloride, polyurethane, polyamide, a polyamide elastomer, and a polyester elastomer can be used.

In addition, in Fig. 3, it is preferable that the distal end of the balloon catheter 10 according to the present invention be provided with the distal end member 15 which has a spherical surface playing a role to assist the catheter to move along a blood vessel and prevents a blood vessel wall from being damaged.

The balloon 1 is foldable so that the balloon 1 can be in a state of being folded around the outer circumference of the inner tube 14 while not being dilated.

Then, the balloon 1 is a foldable balloon having a tubular body with a substantially uniform diameter, in which at least a portion thereof has a cylindrical shape so as to easily dilate a stenosed site of a blood vessel or a body cavity.

Then, the connection portion 4b of the balloon 11 is fixed to the distal end portion of the outer tube 12 in a liquid-tight manner by using an adhesive, performing heat-sealing, and the like.

Similarly, the connection portion 4a is also fixed to the distal end portion of the inner tube 14 in a liquid-tight manner.

As illustrated in Fig. 3, in the balloon 1, a space 112 is formed between the inner surface of the balloon 1 and the outer surface of the inner tube 14 during the dilation.

The space 112 on the proximal end side communicates with the second lumen 120 throughout the entire circumference thereof.

In this manner, since the balloon 1 on the proximal end side is caused to communicate with the second lumen having a relatively large volume, it is easy to inject fluid into the balloon 1 through the second lumen.

As the balloon 1, the balloon which has been described above is used.

In addition, in Fig. 3, the balloon 1 is configured to have a three-layered membrane (outer layer, intermediate layer, and inner layer).

In addition, in order to make it possible to check the position of the tubular membranous body of the balloon 1 through an X-ray contrast, it is preferable to provide one or more X-ray markers 44 on the outer surface of the inner tube 14.

As illustrated in Fig. 3, it is preferable that the X-ray markers 44 be provided in a position closer to the rear end side of the balloon 1 than the portion where the balloon 1 is fixed to the inner tube 14 and a position closer to the distal end side of the balloon 1 than the portion where the balloon 1 is fixed to the outer tube 12. In other words, it is preferable that the X-ray markers 44 be provided in the positions at both of the ends of the tubular membranous body 2 of the balloon 1.

It is preferable for the X-ray markers 44 to be formed of a radiopaque material (for example, gold, platinum, iridium, tungsten, or an alloy of these).

The hub 13 according to the present invention has the opening portion 130 that communicates with the second lumen 120 and forms an injection port as an inlet of a path through which fluid is injected or discharged.

Accordingly, the opening portion 130 plays a role of a flow path and communicates with a portion for supplying and discharging fluid (not illustrated), for example, an Indeflator, a syringe, or a pump.

In this manner, fluid is supplied to the balloon 1 through the opening portion 130 and the second lumen 120, or discharged out of the balloon 1.

In other words, the opening portion 130 and the lumen 120 function as pathways for supplying and discharging drive fluid which cause the balloon 1 to dilate or contract.

As a material for forming the hub according to the present invention, a thermoplastic resin such as polycarbonate, polyamide, polysulfone, polyarylate, and a methacrylate-butylene-styrene copolymer can be preferably used.

### Examples

Hereinafter, specific examples according to the present invention will be described, and the scope of the present invention is not limited thereto.

### (Hereinafter, Examples will be described.)

### "Producing of Balloon"

### (Example 1)

A three-layered tube (expansion magnification of inner diameter: 8 times, expansion magnification in axial direction: 3 times, φ 0.360 mm × 0.485 mm × 0.825 mm × 0.875 mm, each diameter ±0.03 mm) in which a polyamide elastomer (manufactured by EMS-GRIVORY, trade name: Grilflex ELG5660) resin was adopted as an elastomer to be used in the inner layer and the outer layer, and nylon 12 (manufactured by EMS-GRIVORY, trade name: Grilamid L25) was adopted for the intermediate layer was subjected to the coextrusion molding at a temperature of 170°C to 260°C.

Thereafter, blow molding was performed by blowing dry nitrogen into the obtained tube at a temperature of 90°C and a pressure of 3.8 MPa for a certain period, thereby producing a balloon having an outer diameter (nominal diameter) of 3.00 mm, the average wall thickness of 21 µm, and a length of 15 mm.

The average thickness of the membrane of the intermediate layer was 14.5 µm, and the ratio of the intermediate layer was 70%.

The average thickness of the membrane mentioned herein denotes an arithmetic mean in arbitrary positions (6 places) of the balloon, and as a measuring method, Digimatic Indicator manufactured by Mitutoyo Corporation is used to perform the measuring.

### (Example 2)

A three-layered tube (expansion magnification of inner diameter: 8 times, expansion magnification in axial direction: 3 times, φ 0.360 mm × 0.485 mm × 0.805 mm × 0.875 mm, each diameter ±0.03 mm) in which a polyamide elastomer (manufactured by EMS-GRIVORY, trade name: Grilflex ELG5660) resin was adopted as an elastomer to be used in the inner layer and the outer layer, and nylon 12 (manufactured by EMS-GRIVORY, trade name: Grilamid L25) was adopted for the intermediate layer was subjected to the coextrusion molding at a temperature of 170°C to 260°C.

Thereafter, blow molding was performed by blowing dry nitrogen into the obtained tube at a temperature of 90°C and a pressure of 3.8 MPa for a certain period, thereby producing a balloon having an outer diameter (nominal diameter) of 3.00 mm, an average wall thickness of 21 µm, and a length of 15 mm.

The average thickness of the membrane of the intermediate layer measured by a method same as that in (Example 1) was 13.5 µm, and the ratio of the intermediate layer was 65%.

### (Example 3)

A three-layered tube (expansion magnification of inner diameter: 8 times, expansion magnification in axial direction: 3 times, φ 0.360 mm × 0.485 mm × 0.785 mm × 0.875 mm, each diameter ±0.03 mm) in which a polyamide elastomer (manufactured by EMS-GRIVORY, trade name: Grilflex ELG5660) resin was adopted as an elastomer to be used in the inner layer and the outer layer, and nylon 12 (manufactured by EMS-GRIVORY, trade name: Grilamid L25) was adopted for the intermediate layer was subjected to the coextrusion molding at a temperature of 170°C to 260°C.

Thereafter, blow molding was performed by blowing dry nitrogen into the obtained tube at a temperature of 90°C and a pressure of 3.8 MPa for a certain period, thereby producing a balloon having an outer diameter (nominal diameter) of 3.00 mm, the average wall thickness of 21 µm, and a length of 15 mm.

The average thickness of the membrane of the intermediate layer measured by a method same as that in (Example 1) was 12.5 µm, and the ratio of the intermediate layer was 60%.

### (Example 4)

A three-layered tube (expansion magnification of inner diameter: 8 times, expansion magnification in axial direction: 3 times, φ 0.360 mm × 0.485 mm × 0.765 mm × 0.875 mm, each diameter ±0.03 mm) in which a polyamide elastomer (manufactured by EMS-GRIVORY, trade name: Grilflex ELG5660) resin was adopted as an elastomer to be used in the inner layer and the outer layer, and nylon 12 (manufactured by EMS-GRIVORY, trade name: Grilamid L25) was adopted for the intermediate layer was molded at a temperature of 170°C to 260°C.

Thereafter, blow molding was performed by blowing dry nitrogen into the obtained tube at a temperature of 90°C and a pressure of 3.8 MPa for a certain period, thereby producing a balloon having an outer diameter (nominal diameter) of 3.00 mm, the average wall thickness of 21 µm, and a length of 15 mm.

The average thickness of the membrane of the intermediate layer measured by a method same as that in (Example 1) was 11.5 µm, and the ratio of the intermediate layer was 55%.

### (Example 5)

A three-layered tube (expansion magnification of inner diameter: 8 times, expansion magnification in axial direction: 3 times, φ 0.360 mm × 0.485 mm × 0.745 mm × 0.875 mm, each diameter ±0.03 mm) in which a polyamide elastomer (manufactured by EMS-GRIVORY, trade name: Grilflex ELG5660) resin was adopted as an elastomer to be used in the inner layer and the outer layer, and nylon 12 (manufactured by EMS-GRIVORY, trade name: Grilamid L25) was adopted for the intermediate layer was subjected to the coextrusion molding at a temperature of 170°C to 260°C.

Thereafter, blow molding was performed by blowing dry nitrogen into the obtained tube at a temperature of 90°C and a pressure of 3.8 MPa for a certain period, thereby producing a balloon having an outer diameter (nominal diameter) of 3.00 mm, the average wall thickness of 21 µm, and a length of 15 mm.

The average thickness of the membrane of the intermediate layer measured by a method same as that in (Example 1) was 10.5 µm, and the ratio of the intermediate layer was 50%.

### (Example 6)

A three-layered tube (expansion magnification of inner diameter: 8 times, expansion magnification in axial direction: 3 times, φ 0.360 mm × 0.485 mm × 0.675 mm × 0.875 mm, each diameter ±0.03 mm) in which a polyamide elastomer (manufactured by EMS-GRIVORY, trade name: Grilflex ELG5660) resin was adopted as an elastomer to be used in the inner layer and the outer layer, and nylon 12 (manufactured by EMS-GRIVORY, trade name: Grilamid L25) was adopted for the intermediate layer was molded at a temperature of 170°C to 260°C.

Thereafter, blow molding was performed by blowing dry nitrogen into the obtained tube at a temperature of 90°C and a pressure of 3.8 MPa for a certain period, thereby producing a balloon having an outer diameter (nominal diameter) of 3.00 mm, the average wall thickness of 21 µm, and a length of 15 mm.

The average thickness of the membrane of the intermediate layer measured by a method same as that in (Example 1) was 7.5 µm, and the ratio of the intermediate layer was 35%.

### (Comparative Example 1)

As a comparative example, a two-layered balloon in the related art was used.

An inner layer thereof was formed of polyamide, and an outer layer thereof was formed of a polyamide elastomer.

The ratio of the thickness of the membrane of the inner layer formed of polyamide was 84% with respect to the thickness of the overall membrane of the balloon.

### (Comparative Example 2)

As another comparative example, a one-layered balloon in the related art was used.

The material thereof was a material made of a mixture of polyamide elastomers.

### "Evaluation of Balloon"

### (Evaluation of Pressure Resistance and Evaluation of Compliance Characteristics)

The balloons obtained through the examples were subjected to measuring of an amount of change in diameter with respect to pressurization using a compression tester manufactured by Terumo Corporation, thereby calculating the diameter of the balloon under each pressure for the balloons of Examples 1 to 6 and the balloons of Comparative Examples 1 and 2, respectively.

As a method of measuring of an amount of change in diameter with respect to pressurization, similar to the measuring rupture resistance performance, a sample to be measured was immersed in warm water at the temperature of 37°C. When a step of applying a desired internal pressure for one minute and depressurizing to 1 atm is referred to as one cycle, the desired pressure was set as follows such as 1, 6, 8, 10,...20, 21,... atm to repeat the cycle. Then, measuring of the diameter was performed under certain pressures.

Subsequently, having the diameter in the nominal pressure (recommended expansion pressure) reaching the nominal diameter of the balloon as a reference, the expansion rate under certain pressures (that is, ratio of change of compliance or diameter under certain pressures) was calculated.

The result is indicated in Table 1.

**[Table 1]**

| | | Increase of Pressure from Nominal Pressure | | | | | | | | | | |
|---|---|---|---|---|---|---|---|---|---|---|---|---|
| | | (+) 0atm | (+) 2atm | (+) 4atm | (+) 6atm | (+) 8atm | (+) 10atm | (+) 12atm | (+) 14atm | (+) 16atm | (+) 18atm | (+) 20atm |
| Expansion rate % | Intermediate Ratio 70% | 0.00 | 1.01 | 2.00 | 3.01 | 4.03 | 5.32 | 6.64 | 8.09 | 9.75 | 11.45 | 13.53 |
| | Intermediate Ratio 65% | 0.00 | 0.97 | 1.94 | 2.92 | 3.92 | 5.13 | 6.37 | 7.72 | 9.32 | 11.28 | 13.55 |
| | Intermediate Ratio 60% | 0.00 | 0.98 | 1. 98 | 3.00 | 4.02 | 5.32 | 6.63 | 8.13 | 9.99 | 12.35 | 14.82 |
| | Intermediate Ratio 55% | 0.00 | 0.97 | 1.99 | 3.01 | 4.05 | 5.35 | 6.73 | 8.33 | 10.38 | 13.00 | 13.98 |
| | Intermediate Ratio 50% | 0.00 | 1.00 | 2.01 | 3.05 | 4.10 | 5.45 | 6.90 | 8.64 | 10.81 | 13.58 | |
| | Intermediate Ratio 35% | 0.00 | 1.42 | 2.72 | 4.06 | 5.42 | 7.32 | 9.33 | 11.92 | 14.39 | | |
| | Comparative Example 1 | 0.00 | 1.09 | 2.11 | 3.13 | 4.19 | 5.24 | 6.61 | 8.09 | 9.86 | 12.06 | |
| | Comparative Example 2 | 0.00 | 3.00 | 5.00 | 7.00 | 9.00 | 11.00 | 14.00 | | | | |

The expansion rate (%) under certain pressures denotes that after the diameter in each nominal pressure is subtracted from the diameter in each of the certain pressures of the balloon, the value divided by the diameter in the nominal pressure is multiplied by 100.

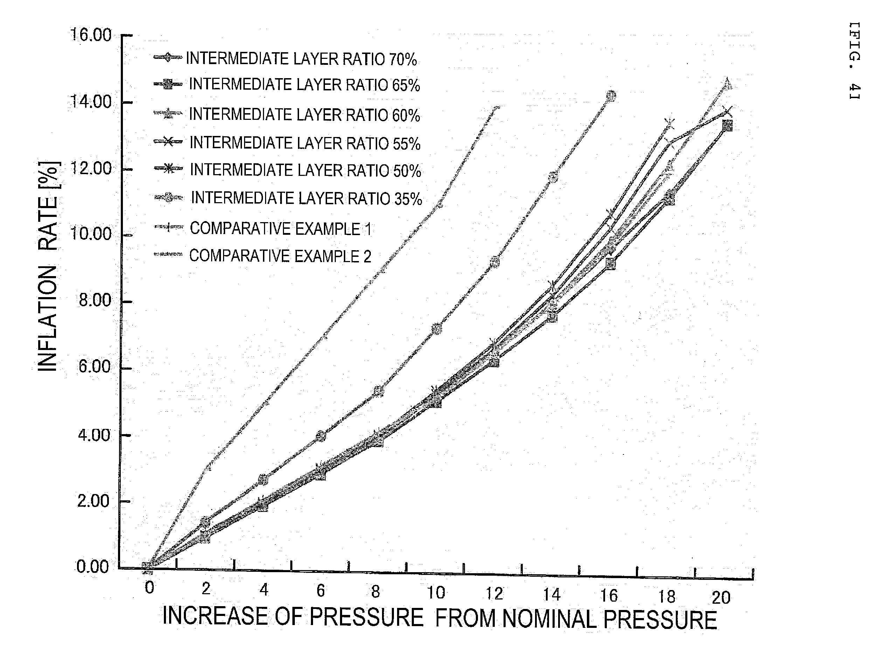

Table 1 is expressed as a graph in Fig. 4.

The balloons of Examples 1 to 6 and the balloon of Comparative Example 1 exhibited the diminished compliance performance and the rupture resistance performance even when these are under high inflation pressure.

While lowering the intermediate layer ratio as indicated in Fig. 4, in the balloons of Examples 1 to 5, the expansion rates in the nominal pressure plus approximately 13 atm were equal to or less than 8%, and in the balloon of Example 6, the radial expansion rate in the nominal pressure plus approximately 13 atm was equal to or less than 11%, thereby realizing the diminished compliance.

Accordingly, it has been understood that the balloon has dilation performance suitable for the post-expansion of a stent.

### (Evaluation of Flexibility)

The balloons obtained through the examples were subjected to measurement of bending elastic moduli of three-point bending by using a compact tabletop load tester (MODEL 1305N) manufactured by Aikoh Engineering Co., Ltd.

The measurement was performed under sample holding intervals of 25.4 mm, a push-in length of 2 mm, and a push-in speed of 5 mm/min, thereby respectively calculating the bending elastic moduli (kgf/cm²) of the balloons in Examples 1 to 6 and Comparative Examples.

The results are indicated in Tables 2 and 3.

**[Table 2]**

| | Bending Elastic Modulus (kgf/cm²) |
|---|---|
| Comparative Example 1 | 11000 |
| Example 1 (Intermediate layer ratio 70%) | 9100 |
| Example 4 (Intermediate layer ratio 55%) | 7400 |
| Example 6 (Intermediate layer ratio 35%) | 4567 |
| Comparative Example 2 | 4900 |

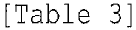

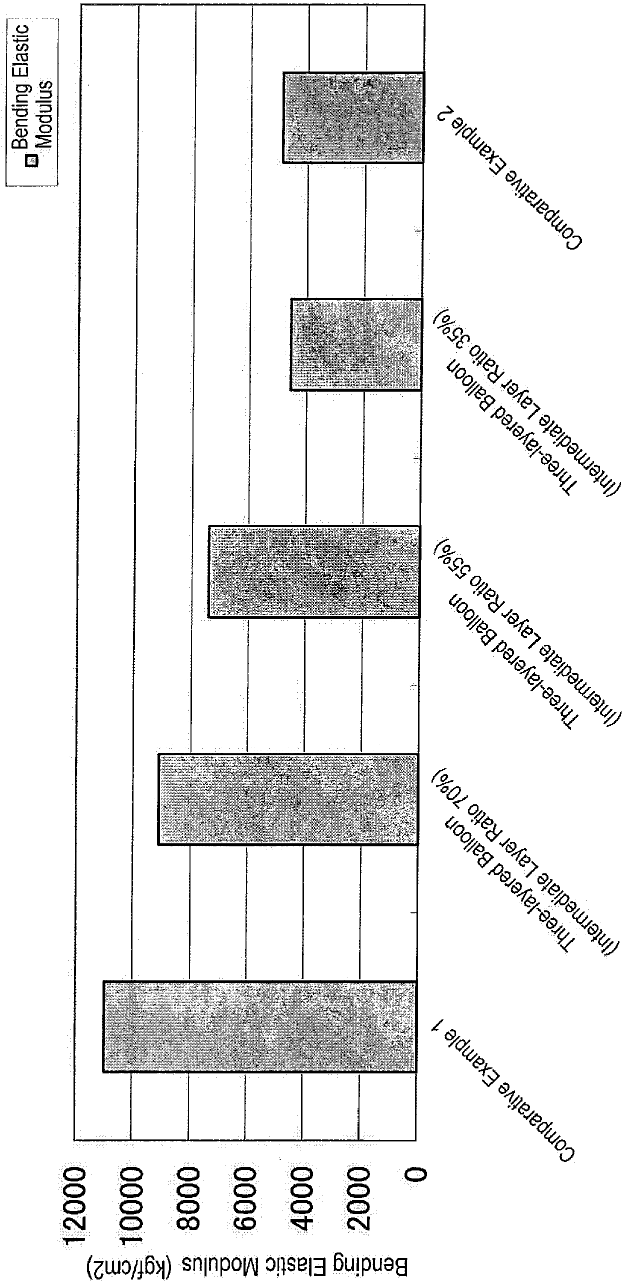

According to Tables 2 and 3, when the ratio of the average thickness of the intermediate layer according to the present invention is within 35% to 70%, the ratio belongs within the range of flexibility of the balloon in the related art, thereby being considered to be favorable in the passing characteristics.

Particularly, it has been learned that Example 6 in which the average thickness of the intermediate layer is 35% with respect to the thickness of the balloon in its entirety of the present invention excels in the passing characteristics in particular.

### Reference Signs List

- 1: balloon
- 2: tubular membranous body
- 3a, 3b: opening portion
- 4a, 4b: connection portion with respect to catheter
- 5a, 5b: tapered portion
- 6: inner layer containing elastomer
- 7: intermediate layer containing non-elastomer
- 8: outer layer containing elastomer
- 101: catheter body
- 12: outer tube
- 13: hub
- 14: inner tube
- 18: first opening portion (guide wire port)
- 130: second opening portion (injection port)
- 120: second lumen
- 150: first lumen

## Claims

1. A catheter balloon comprising:
a membranous body that can dilate and contract by fluid supplied from a catheter,
wherein the membranous body includes an intermediate layer which contains a non-elastomer, an outer layer which is arranged on an outer surface of the intermediate layer and contains an elastomer, and an inner layer which is arranged on an inner surface of the intermediate layer and contains an elastomer, and
wherein the average wall thickness of the intermediate layer is within 30% to 70% of the average wall thickness of the balloon in its entirety.

2. The catheter balloon according to Claim 1,
wherein the balloon has an radial expansion rate of approximately 6% to approximately 16% within an inflation pressure range from a nominal pressure to the nominal pressure plus approximately 13 atm.

3. The catheter balloon according to any one of Claim 1 or 2,
wherein the balloon is molded by inflating a tube which is obtained through three-layered coextrusion molding for molding a balloon to six times to nine times of an initial inner diameter in the radial direction and to twice to four times of a stretching ratio in an axial direction.

4. The catheter balloon according to any one of Claims 1 to 3,
wherein the balloon has rupture resistance performance in the inflation pressure of the nominal pressure plus approximately 13 atm.

5. The catheter balloon according to any one of Claims 1 to 4,
wherein a material for the outer layer arranged on the outer surface of the intermediate layer and a material for the inner layer arranged on the inner surface of the intermediate layer are the same with each other.

6. The catheter balloon according to any one of Claims 1 to 5,
wherein the intermediate layer is formed of polyamide.

7. The catheter balloon according to any one of Claims 1 to 6,
wherein each of the inner layer and the outer layer is formed of an polyamide elastomer.

8. A balloon catheter for widening a blood vessel, comprising:
an inner tube that has a first lumen of which the distal end is open;
an outer tube that is provided coaxially with the inner tube, has the distal end in a position which recedes for a predetermined length from the distal end of the inner tube, and forms a second lumen between the an outer surface of the inner tube and itself; and
a foldable balloon of which a distal end portion is fixed to the inner tube, a proximal end portion is fixed to the outer tube, and the inside thereof communicates with the second lumen,
wherein the balloon corresponds to the catheter balloon according to any one of Claims 1 to 7.
